# EUROPEAN PATENT SPECIFICATION

(11) **EP 3 941 633 B1**
(45) Date of publication and mention of the grant of the patent: **28.12.2022**
(21) Application number: 20715172.1
(22) Date of filing: 18.03.2020
(51) Int. Cl.: B01L 3/02, B01L 3/00, B01J 19/00, C12M 3/06

(54) **METHOD AND SYSTEM FOR MANUFACTURING A MICROFLUIDIC ARRANGEMENT, METHOD OF MANUFACTURING A LIQUID, METHOD OF PERFORMING A BIOLOGICAL ASSAY**
VERFAHREN UND SYSTEM ZUR HERSTELLUNG EINER MIKROFLUIDISCHEN ANORDNUNG, VERFAHREN ZUR HERSTELLUNG EINER FLÜSSIGKEIT, VERFAHREN ZUR DURCHFÜHRUNG EINES BIOLOGISCHEN TESTS
PROCÉDÉ ET SYSTÈME DE FABRICATION D'UN AGENCEMENT MICROFLUIDIQUE, PROCÉDÉ DE FABRICATION D'UN LIQUIDE, PROCÉDÉ DE RÉALISATION D'UN DOSAGE BIOLOGIQUE

(30) Priority: 19.03.2019 GB 201903743
(43) Date of publication of application: 26.01.2022
(73) Proprietor: Oxford University Innovation Limited, Oxford, OX2 0JB (GB)
(72) Inventor: FEUERBORN, Alexander, Oxford Oxfordshire OX1 3RE (GB); WALSH, Edmond, Oxford Oxfordshire OX2 0ES (GB)
(74) Representative: J A Kemp LLP
(86) International application number: PCT/GB2020/050716
(87) International publication number: WO 2020/188277

(56) References cited:
- EP-A2- 0 164 813
- WO-A1-2017/064514
- CRISTIAN SOITU ET AL: "Microfluidic chambers using fluid walls for cell biology", PROCEEDINGS OF THE NATIONAL ACADEMY OF SCIENCES, vol. 115, no. 26, 12 June 2018 (2018-06-12), pages E5926-E5933, XP055690231, US ISSN: 0027-8424, DOI: 10.1073/pnas.1805449115

## Description

The invention relates to creating a microfluidic arrangement by dividing a body of a first liquid into a plurality of sub-bodies that are separated from each other by a second liquid. The sub-bodies can be used to provide isolated samples, or microfluidic circuits with liquid walls, containing material to be investigated, such as living cells or other biological material.

Microwell plates are widely used for studies involving biological material. Miniaturisation of the wells allows large numbers of wells to be provided in the same plate. For example, plates having more than 1000 wells, each having a volume in the region of tens of nanolitres, are known. Further miniaturisation is difficult, however, due to the intrinsic need to provide solid walls that separate the wells from each other. A further obstacle to miniaturisation is the difficulty of adding liquids to small wells defined by physical walls. Microwell plates also lack flexibility because the size of the wells and the number of wells per plate is fixed. Furthermore, biological and chemical compatibility can be limited by the need to use a material that can form the structures corresponding to the wells in an efficient manner.

WO 2017/064514 A1 discloses an alternative approach in which individual bodies of aqueous liquid are separated from each other by an immiscible fluorocarbon. This approach overcomes many of the problems with microwell plates having solid walls, but it would be desirable to improve the speed and/or reliability with which the microfluidic arrangements can be formed.

Microwell plates and/or microfluidic arrangements having liquid walls are commonly used to perform experiments involving biological matter such as living cells. These experiments often aim to imitate, or would benefit from imitating, environment conditions within the living body. This can be difficult to achieve throughout the experiment, however, particularly where the microwell plate or microfluidic arrangement needs to be moved between different locations, such as between an incubator and other environments. It is difficult, for example, to avoid exposing the cells to unrealistically high oxygen levels due to the relatively high level of oxygen in air compared to conditions within the body.

CRISTIAN SOITU ET AL, "Microfluidic chambers using fluid walls for cell biology", PROCEEDINGS OF THE NATIONAL ACADEMY OF SCIENCES, US, (20180612), vol. 115, no. 26, doi: 10.1073/pnas.1805449115, ISSN 0027-8424, pages E5926 - E5933 discloses a method for creating microfluidic environments by reshaping fluids on a substrate. For applications in cell biology, cell media on a virgin Petri dish is overlaid with an immiscible fluorocarbon. A hydro-phobic/fluorophilic stylus then reshapes the media into any pattern by creating liquid walls of fluorocarbon. EP 0 164 813 A2 relates to a method of cultivating animal and plant cells wherein a fluorinated oil Fluorinert^{™} FC-40 having dissolved oxygen in it is circulated into a fermentor.

It is an object of the invention to at least partially address one or more of the issues discussed above.

According to an aspect of the invention, there is provided the method of manufacturing a microfluidic arrangement of claim 1.

Thus, a method is provided in which one or more walls of an immiscible second liquid are created and used to hold a first liquid in a modified shape. In some embodiments, the method is implemented in such a way that at least one sub-body of first liquid is separated from the rest of the first liquid by the second liquid. The second liquid acts as a liquid wall, allowing a microfluidic arrangement to be formed in a highly flexible manner and without the disadvantages associated with traditional solid wall alternatives. The treatment of the second liquid by flowing gas through the second liquid improves the fidelity and/or reliability with which the microfluidic arrangement is formed. In the absence of this treatment, it is found that the step of causing the second liquid to move through the first liquid and into contact with the substrate along the selected path is difficult to achieve for many first liquid compositions relevant for biological experiments (e.g. containing proteins/additives for cells) without liquid bridges forming across portions of the selected path. The liquid bridges link together sub-bodies that are supposed to be isolated from each other, thereby leading to incorrect operation of the microfluidic arrangement. The treatment of the second liquid prevents this undesirable effect.

In an embodiment, the gas flowed through the second liquid comprises less than 20% by volume of oxygen. Saturating the liquid with a gas or mixture of gases that has a lower proportion of oxygen than in air may help to emulate conditions within the body more accurately than without the saturation, even where the microfluidic arrangement is exposed directly to atmospheric conditions.

Embodiments of the invention will now be described, by way of example only, with reference to the accompanying drawings in which corresponding reference symbols indicate corresponding parts, and in which:
Figure 1 is a schematic side view of an apparatus for treating a second liquid and providing the second liquid to a microfluidic arrangement;
Figure 2 is a schematic side view of a continuous body of a first liquid on a substrate with a second liquid in direct contact with the first liquid and covering the first liquid;
Figure 3 is a schematic side view of the arrangement of Figure 2 during dividing of the continuous body of the first liquid by pumping a separation fluid out of a distal tip of an injection member;
Figure 4 is a schematic top view of the arrangement of Figure 3;
Figure 5 is a schematic side view showing a subsequent step of further dividing a sub-body;
Figure 6 is a schematic top view of a portion of a microfluidic arrangement formed using the steps of Figures 3-5 showing a closed loop of the selected path surrounding and in contact with a boundary of a sub-body;
Figure 7 is an image of a portion of a microfluidic arrangement formed using a method, not forming part of the claimed invention, in which the second liquid is not treated, showing uneven and broken boundary lines between different sub-bodies of a first liquid having a common cell culture medium composition, indicative of formation of liquid bridges;
Figure 8 is an image of a portion of a microfluidic arrangement formed using the same method as in Figure 7 except that the second liquid was treated prior to separation of the sub-bodies of first liquid;
Figure 9 depicts a dividing scheme in which a continuous body of the first liquid is divided into parallel elongate strips in a first step, wherein each strip is subsequently divided into a plurality of sub-bodies;
Figure 10 depicts a dividing scheme in which a continuous body of the first liquid is divided to form at least one sub-body comprising a conduit connected to at least one reservoir; and
Figure 11 depicts an apparatus for manufacturing a microfluidic arrangement according to embodiments of the disclosure involving pumping of separation fluid out of a distal tip of an injection member.

The figures are provided for explanatory purposes only and are not depicted to scale in order to allow constituent elements to be visualised clearly. In particular, the width of the receptacle providing the substrate relative to the depth of the first and second liquids will typically be much larger than depicted in the drawings.

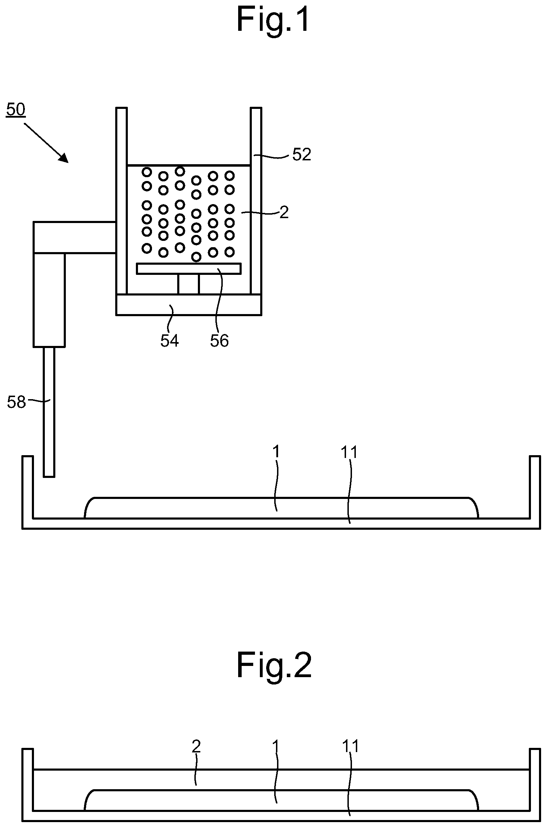

Figure 1 depicts a liquid treatment apparatus 50 for treating a liquid. The liquid is a second liquid 2 for use in manufacturing a microfluidic arrangement. The apparatus 50 is configured to flow a gas through the second liquid 2. The flow of gas through the second liquid 2 changes the properties of the second liquid 2 such that when the second liquid 2 is used to form the microfluidic arrangement, the process of manufacturing the microfluidic arrangement proceeds with higher fidelity and/or reliability. Without wishing to be bound by theory, it is thought that the flowing of the gas through the second liquid 2 causes an increase in a level of saturation of the second liquid 2 and that it is this change in composition of the second liquid 2 that causes the improved performance of the second liquid 2. The flowing of gas through the second liquid 2 may be referred to as treating the second liquid 2.

Arranging for a gas to flow through the second liquid 2 can be achieved in multiple ways. In the particular example shown in Figure 1, the apparatus 50 comprises a reservoir 52 for holding the second liquid 2 while a pump 54 causes the gas to flow through the second liquid 2. In this example, the pump 54 is coupled to an outlet head 56 comprising plural outlet orifices facing upwards into the reservoir 52. The pump 54 pumps gas (air in this example) from outside of the reservoir 52 into the second liquid 2 in the reservoir 52 through the outlet orifices of the outlet head 56 causing bubbles of the gas to flow upwards through the second liquid 2, driven by buoyancy. It is not essential that the gas is air. In other embodiments, a gas source comprising gas having a different composition to air may be coupled to the outlet head 56. Different examples of gas compositions are described in further detail below.

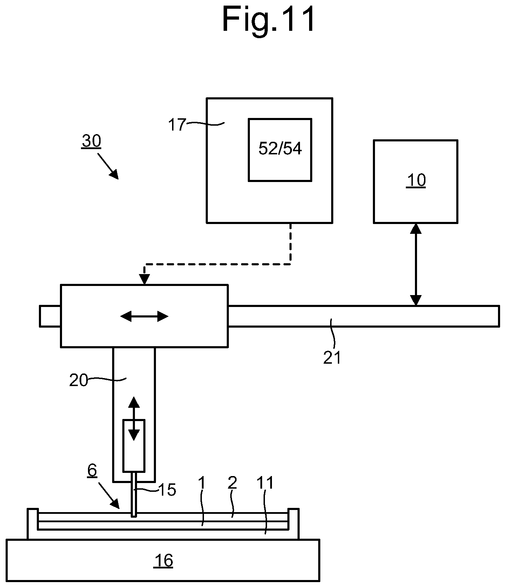

In the embodiment shown, a supply conduit 58 is provided for dispensing the second liquid 2 from the reservoir 52 to a dispensing location when the second liquid 2 needs to be provided during manufacture of a microfluidic arrangement. The liquid treatment apparatus 50 of Figure 1 is depicted as a self-contained unit, but this is not essential. In other embodiments, such as described below with reference to Figure 11, the liquid treatment apparatus 50 may be integrated into an apparatus 30 for manufacturing a microfluidic arrangement, and thereby share elements such as a gantry system 21 and a processing head 20 in order to position the supply conduit 58 appropriately to dispense the second liquid 2.

Figure 1 shows the supply conduit 58 positioned over a lateral portion of a substrate 11, representing an early stage in the manufacture of a microfluidic arrangement. A continuous body of a first liquid 1 is provided. The first liquid 1 is in direct contact with the substrate 11. The first liquid 1 is aqueous (e.g. an aqueous solution). In a class of embodiments, the first liquid 1 comprises, consists essentially of, or consists of a cell culture medium (which may be referred to as growth medium or culture medium). Cell culture medium comprises a liquid containing components (e.g. various proteins/food) for supporting survival and/or proliferation of cells. Examples include Dulbecco's Modified Eagle Medium (DMEM) or Roswell Park Memorial Institute medium (RPMI) with added serum, for example around 10% serum, for example around 10|% Fetal Bovine Serum (FBS). Many nowadays use serum free media, where the FBS or other animal derived substances are not used. Cell culture medium can in general be a liquid or a gel (which typically contains liquid). For example, agar-plates for bacteria comprise a gel formed from a liquid medium. Mostly, however, eukaryotic cells are grown in liquid cell culture medium. The liquid cell culture medium is a source of energy (often in the form of glucose), amino acids, vitamins and some trace elements. Supplements may be added to those media, such as FBS, which supports a breadth of different cells. The precise composition of FBS is not known. FBS is not a chemically defined mix because it comes from a biological source. FBS is known, however, to contain at least hormones, lipids, proteins, and growth-factors which all regulate cell behaviour - i.e., they make cells grow and duplicate, regulate membrane physiology, etc. In many instances it would be beneficial to avoid using FBS. This is because 1) FBS is undefined and therefore variable, and 2) FBS comes from animals and could carry pathogens. The latter factor is important for industries that produce drugs, where it may be necessary to ensure that no components used in a whole manufacturing pipeline have animal-origin. As a result, alternative supplements are now available which are either fully chemically defined and animal free or, if not animal free, at least better defined than serum.

The cell culture medium may be provided as a coating and/or in gel form. Gels are formed mostly from liquid but are constrained to have a degree of rigidity by a solid three-dimensional network spanning the volume of the liquid and ensnaring the liquid through surface tension effects. The first liquid 1 may thus form part of a gel in the case where the cell culture medium is provided as a gel. In an embodiment, cell culture medium comprises a coating arranged to coat a naked polystyrene dish with a layer of molecules that support cell growth and attachment. Various coatings of this type may be used. The coatings may, for example, be derived from a biological source and therefore vary in makeup or be defined. The coatings may thus comprise complex mixtures of components, or a particular ("pure") molecule. Examples of coatings include Coring^{®} Matrigel^{®} Matrix (a relatively complex mixture) and Laminin (a particular molecule). In some embodiments, the coating is formed by applying the cell culture medium to a dish, waiting for some time, and then remove the bulk of the liquid to leave behind a thin layer. The remaining thin layer may then be rinsed, overlaid with the second liquid 2, and processed further to provide sub-bodies of the first liquid 1 as described elsewhere herein.

The second liquid 2 is immiscible with the first liquid 1. In an embodiment, the second liquid 2 comprises, consists essentially of, or consists of a fluorocarbon. In an embodiment, the fluorocarbon comprises a fluorocarbon that has a high enough permeability to allow exchange of vital gases between any cells provided in the first liquid 1 and the surrounding atmosphere through the layer of the second liquid 2. In an embodiment, the fluorocarbon comprises a transparent fully fluorinated liquid of density about 1.8555 g/ml that is widely used in droplet-based microfluidics, such as FC-40 (Fluorinert^{™} Electronic Liquid FC-40 sold by 3M) or similar. The manufacture of the microfluidic arrangement comprises dispensing the second liquid 2 onto the substrate 11 through the supply conduit 58 to provide the arrangement shown in Figure 2. As depicted in Figure 2, the second liquid 2 is thereby provided in direct contact with the first liquid 1. The second liquid 2 treated by the flowing of gas through the second liquid 2 before the second liquid 2 is brought into contact with the first liquid 1 (i.e. prior to the providing of the second liquid in direct contact with the continuous body of first liquid).

The second liquid 2 is immiscible with the first liquid. The continuous body of the first liquid 1 is formed on the substrate 11 before the second liquid 2 is brought into contact with the first liquid 1. In other embodiments, not forming part of the claimed method, the continuous body of the first liquid 1 is formed after the second liquid 2 is provided (e.g. by injecting the first liquid 1 through the first liquid 2). In embodiments in which the microfluidic arrangement is to be used for testing samples of biological material, and in the context of the claimed method, the continuous body of the first liquid 1 will normally be formed before the second liquid 2 is provided. The second liquid 2 covers the first liquid 1. The first liquid 1 is thus completely surrounded and in direct contact exclusively with a combination of the second liquid 2 and the substrate 11. At this point in the method the first liquid 1 is not in contact with anything other than the second liquid 2 and the substrate. Typically, the substrate 11 will be unpatterned (neither mechanically nor chemically), at least in the region in contact with (typically underneath) the continuous body of the first liquid 1. In an embodiment, the continuous body of the first liquid 1 is in direct contact exclusively with a substantially planar portion of the substrate 11 and the second liquid 2.

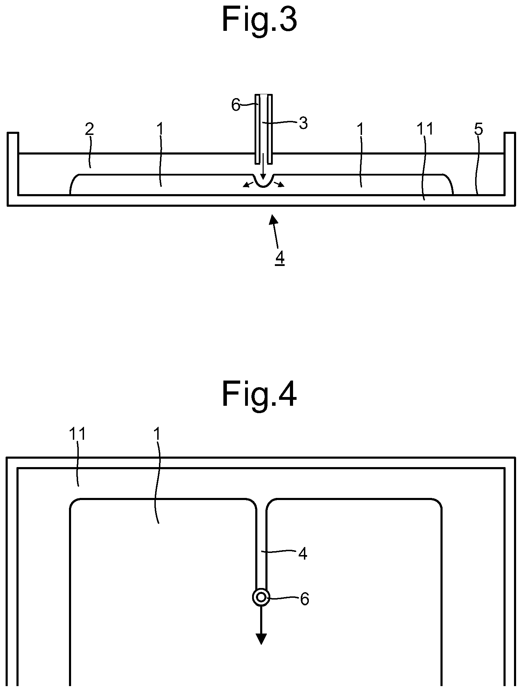

In a subsequent step, an example implementation of which is depicted in Figure 3, a separation fluid 3 is propelled through at least the first liquid 1 (and optionally also through a portion of the second liquid 2, as shown in the example of Figure 3) and into contact with the substrate 11 along all of a selected path 4 on the surface 5 of the substrate 11. The selected path 4 consists of a portion of the surface area of the surface 5 of the substrate 11. The selected path 4 thus has a finite width. Portions of the selected path 4 may be substantially elongate and interconnected, the selected path thereby forming a network or web-like pattern. The separation fluid 3 is immiscible with the first liquid 1. The separation fluid 3 displaces the first liquid 1 away from the selected path 4. In the example shown, the displacement of the first liquid 1 away from the selected path 4 is achieved without any solid member contacting the selected path directly (e.g. by dragging a tip of the solid member over the surface of the substrate 11) and without any solid member contacting the selected path via a globule of liquid held at a tip of the solid member (e.g. by dragging the globule of liquid, held stationary relative to the tip, over the substrate 11). In other embodiments, the displacement of the first liquid 1 away from the selected path 4 is achieved by dragging a tip of a solid member over the surface of the substrate 11. In other embodiments, the displacement of the first liquid 1 away from the selected path 4 is achieved by dragging a globule of liquid, held stationary relative to the tip of a solid member, over the substrate 11. Whichever method is used, the displacement of the first liquid 1 away from the selected path involves movement of the second liquid 2 through the first liquid 1 and into contact with the substrate 11 along all of the selected path 4. The surface area defined by the selected path 4 represents a portion of the surface area of the substrate 11 in which the first liquid 1 has been displaced away from contact with the substrate 11.

In the embodiment of Figure 3, the separation fluid 3 is propelled onto the selected path 4 from a lumen in a distal tip 6 of an injection member while the distal tip 6 is scanned over the substrate 11. No contact is therefore made in this embodiment between the distal tip 6 and the selected path 4 during at least a portion of the selected path 4. The momentum of the separation fluid 3 is sufficient to force the first liquid 1 to be displaced away from the selected path 4. In an embodiment, the separation fluid 3 is pumped continuously out of the distal tip for at least a portion of the selected path. In the embodiment shown in Figure 3, the separation fluid 3 is pumped out of the distal tip 6 in a direction that is substantially perpendicularly to the selected path 4 at the location of the distal tip 6. In other embodiments, the distal tip 6 may be tilted so as to pump the separation fluid 3 towards the selected path 4 at an oblique angle relative to the selected path 4.

As depicted for example in Figures 4-6, the selected path 4 is such that one or more walls of second liquid 2 are formed that modify a shape of the continuous body of first liquid 1. The walls of second liquid 2 are formed from the second liquid 2 in contact with, and extending upwards from, the selected path 4. The geometry of the walls of second liquid 2 is therefore defined by (e.g. substantially the same as) the geometry of the selected path 4. In some embodiments, the continuous body of first liquid 1 remains a single continuous body of first liquid 1 after the modification of the shape of the continuous body of first liquid 1 by the one or more walls of second liquid 2. In other embodiments, as in the examples shown, the continuous body of the first liquid 1 is divided into a plurality of sub-bodies 7 of the first liquid 1. Each sub-body 7 is separated from each other sub-body 7 by the second liquid 2. Thus, when the first liquid 1 is displaced away from the selected path 4 by the propelled separation fluid 3, the second liquid 2 moves into contact with the selected path 4 and remains stably in contact with the selected path 4. A pinning line (associated with interfacial forces) stably holds the plurality of sub-bodies 7 of the first liquid 1 separated from each other by the second liquid 2. The plurality of sub-bodies 7 may comprise a single useful sub-body 7 and a remainder of the continuous body of the first liquid 1 (which may be considered as another sub-body) or may comprise plural useful sub-bodies (e.g. plural reservoirs for receiving reagents etc.), optionally together with any remainder of the continuous body of the first liquid 1.

Thus, the one or more walls of second liquid 2 define features of the microfluidic arrangement. In an embodiment, the features comprise one or more closed features, thereby defining sub-bodies of the first liquid 1 formed by dividing the continuous body of first liquid 1 into a plurality of sub-bodies of the first liquid 1 via the one or more walls of second liquid 2. Each sub-body is separated from each other sub-body by the second liquid 2. Such a plurality of sub-bodies may comprise a single useful sub-body and a remainder of the continuous body of the first liquid 1 (which may be considered as another sub-body) or may comprise plural useful sub-bodies (e.g. plural reservoirs for receiving reagents etc.), optionally together with any remainder of the continuous body of the first liquid 1. In an embodiment, the features comprise one or more open features. The open features may include, for example, open-ended flow conduits or open-ended chambers. The flow conduits may comprise portions of the first liquid 1 that are constrained by the one or more walls of second liquid to adopt an elongate shape (e.g. surrounded laterally and from above by the second liquid). The continuous body of first liquid 1 may thus remain a single continuous body of first liquid 1 after the modification of the shape of the continuous body of first liquid 1 by the one or more walls of second liquid 2. The continuous body of first liquid 1 is continuous in that every point in the continuous body of first liquid is connected to every other point in the continuous body of first liquid 1 along an uninterrupted path going exclusively through the first liquid 1. The continuous body of first liquid 1 is not divided into isolated sub-bodies in embodiments of this type.

The separation fluid 3 may comprise one or more of the following: a gas, a liquid, a liquid having the same composition as the second liquid 2, a portion of the second liquid 2 provided before the propulsion of the separation fluid 3 through the first liquid 1. In some embodiments, the separation fluid 3 is propelled onto the selected path 4 on the substrate 11 from a lumen (e.g. by continuously pumping the separation fluid 3 out of the lumen, optionally at a substantially constant rate) in a distal tip 6 of an injection member while providing relative movement between the distal tip 6 and the substrate 11 (e.g. by scanning the distal tip 6 over or under the substrate 11 along a path corresponding to the selected path 4), with some first liquid 1 and, optionally, second liquid 2, between the distal tip 6 and the substrate 11. Either or both of the distal tip 6 and the substrate 11 may be moved in order to provide the relative movement between them. In some embodiments of this type, the distal tip 6 is moved through both of the second liquid 2 and the first liquid 1 while propelling the separation fluid 3 onto the selected path 4 on the substrate 11, for at least a portion of the selected path 4. The distal tip 6 is thus held relatively close to the substrate 11. In such embodiments, the movement of the distal tip 6 and the flow of the separation fluid 3 towards the substrate 11 both act to displace the first liquid 1 away from the substrate 11, allowing the second liquid 2 to move into the volume previously occupied by the first liquid 1. In an embodiment, this process is facilitated by arranging for at least a portion of the distal tip 6 to be more easily wetted by the second liquid 2 than by the first liquid 1. In this way, it is energetically more favourable for the second liquid 2 to flow into the region behind the moving distal tip 6 and thereby displace the first liquid 1 efficiently. Preferably the substrate 11 is also configured so that it is more easily wetted by the second liquid 2 than by the first liquid 1, thereby energetically favouring contact between the second liquid 2 and the substrate 11 along the selected path 4. This helps to maintain a stable arrangement in which the sub-bodies 7 are separated from each other by second liquid 2 in contact with the selected path 4. In other embodiments, an example of which is shown in Figure 3, the distal tip 6 is moved through the second liquid 2 but not the first liquid 1 while propelling the separation fluid 3 onto the selected path 4 on the substrate 11, for at least a portion of the selected path 4. The distal tip 6 is thus held further away from the substrate 11. This approach helps to avoid detachment of droplets of the first liquid 1 from the substrate 11 caused by the pumping of the separation fluid 3 against the substrate 11.

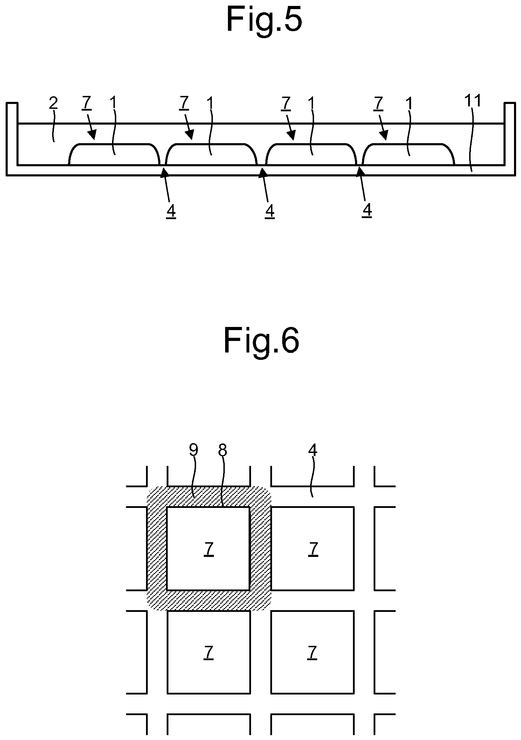

Figures 3 and 4 depict movement of a distal tip 6 through the second liquid 2 but not the first liquid 1 in a horizontal direction, parallel to a plane of the substrate 11 in contact with (typically underneath) the first liquid 1. Separation fluid 3 is pumped from the distal tip 6. The vertical arrow exiting the distal tip 6 in Figure 3 schematically represents a pumped flow of the separation fluid 3. Arrows within the first liquid 1 in Figure 3 schematically represent movement of the first liquid 1 away from the region above a portion of the selected path 4, which will eventually allow the second liquid 2 to contact the substrate 11 along the selected path 4. In Figure 3, the movement of the distal tip 6 is into the page. In Figure 4, the movement is downwards. In an embodiment, the distal tip 6 is maintained at a constant distance from the substrate 11 while the distal tip 6 is being moved through the second liquid 2. When completed, the process of Figures 3 and 4 will result in the continuous body of the first liquid 1 of Figures 1 and 2 being divided into two sub-bodies. The process can be repeated and/or performed in parallel to create the desired number and size of individual sub-bodies 7. The pumping of the separation fluid 3 is optionally stopped and started between movement of the distal tip 6 over different portions of the selected path, or the pumping may continue as the distal tip moves from the end of one portion of the selected path to the start of the next portion of the selected path. Figure 5 depicts the result of repeating the steps of Figures 3 and 4 to create three parallel lines of a selected path 4 (with the pumping of the separation fluid 3 being optionally stopped and started between formation of each of the three parallel lines, or the pumping may continue while the distal tip moves from the end of one parallel line to the start of the next parallel line). By repeating the process in the orthogonal direction 16 square sub-bodies 7 could be provided. In practice, many 100s or 1000s of sub-bodies 7 could be provided in this manner. The inventors have demonstrated for example that the approach can be used routinely to obtain a square array of sub-bodies having a pitch of less than 100 microns. This is considerably smaller than would be possible using standard microwell plate manufacturing techniques.

As depicted for one of the sub-bodies 7 in Figure 6, the selected path 4 is such that, for each of one or more of the sub-bodies 7, a sub-body footprint represents an area of contact between the sub-body 7 and the substrate 11 and all of a boundary 8 of the sub-body footprint is in contact with a closed loop 9 of the selected path 4 (an example of which is depicted by hatching in Figure 6) surrounding the sub-body footprint. The closed loop 9 of the selected path 4 is defined as any region that represents a portion of the surface area of the substrate 11 that forms part of the selected path 4, that forms a closed loop, and that is in contact with the boundary 8 of sub-body 7 along all of the boundary 8 of the sub-body 7. The first liquid 1, second liquid 2 and substrate 11 are configured (e.g. by selecting their compositions) such that each boundary 8 of a sub-body footprint that is all in contact with a closed loop 9 of the selected path 4 is pinned in a static configuration by interfacial forces, with the first liquid 1 and second liquid 2 remaining in liquid form. Thus, interfacial forces, which may also be referred to as surface tension, establish pinning lines that cause the sub-body footprints to maintain their shape. The stability of the sub-bodies 7 formed in this way is such that liquid can be added to or removed from each sub-body 7, within limits defined by the advancing and receding contact angles along the boundary 8, without changing the sub-body footprint. In some embodiments the boundary 8 of the sub-body footprint that is all in contact with the closed loop 9 of the selected path 4 is made continuously (i.e. in a single process without interruption), and in the embodiment illustrated in Figure 6 it is made in four separate steps.

In some embodiments, the separation fluid 3 comprises a portion of the second liquid 2 and the portion of the second liquid 2 is propelled towards the selected path 4 by locally coupling energy into a region containing or adjacent to the portion of the second liquid 2 to be propelled towards the selected path 4 on the substrate 11. The energy coupling may comprise locally generating heat or pressure. The energy may cause expansion, deformation, break-down, ablation or cavitation of material that results in a pressure wave being transmitted towards the portion of the second liquid 2 to be propelled. In some embodiments, the coupling of energy is implemented using a focussed beam of a wave such as electromagnetic radiation or ultrasound. The coupling of energy may occur at or near a focus of the beam.

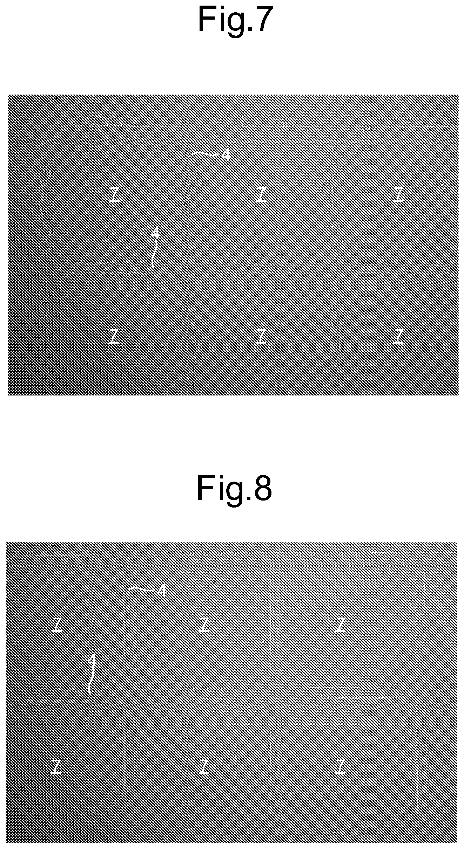

Figures 7 and 8 depict the effect of the treatment of the second liquid 2 when manufacturing a microfluidic arrangement of the type described above with reference to Figures 3-6. Figure 7 depicts the result of using the method without treating the second liquid 2. Figure 8 depicts the result of using the method with treated second liquid 2. In both cases, the first liquid 1 comprised an aqueous solution (containing cell culture medium with 10 % serum) and the second liquid 2 comprised FC-40 (Fluorinert^{™} Electronic Liquid FC-40 sold by 3M^{™}). The grid-like structure shows square regions 7 that each correspond to one intended isolated sub-body 7, nominally separated by closed loop strips making up the selected region 4 where the second liquid 2 is supposed to have been brought continuously into connection with the substrate 11 in such a way as to perfectly isolate each sub-body 7 of first liquid 1 from every other sub-body 7 of first liquid 1. In the example of Figure 7, the process has not worked successfully. Uneven and broken boundary lines between different sub-bodies 7 are observed, indicative of the existence of multiple liquid bridges connecting together sub-bodies 7 that are supposed to be isolated from each other. The effects are seen for a wide range of compositions of first liquid 1 suitable for cell culture, including combinations of DMEM or RPMI with serum, for example around 10% serum, for example around 10% FBS.

In Figure 8, by contrast, the walls of second liquid 2 are seen to be properly formed over all of the region of the substrate 11 corresponding to the selected region 4. All of the sub-bodies 7 of first liquid 1 are thus fully isolated from each other by the second liquid 2 and the microfluidic arrangement can operate as intended.

The above effect has been found to occur for a variety of different liquids commonly used in microfluidics, including various fluorocarbons, including FC-40 and HFE7500 (3M^{™} Novec^{™} Engineering Fluid). The effect has also been found to occur for a variety of different gases used for performing the flowing of gas through the second liquid 2. Furthermore, the effect has been shown to be fully reversible by degassing the second liquid 2 using sonication. Use of the sonicated second liquid 2 to form the microfluidic arrangement results again in the situation of Figure 7 where sub-bodies are no longer properly separated from each other.

In an embodiment, the gas consists of air. Using air has been found to be effective and air is readily available. The air can simply be pumped from an environment rather than having to provide a special gas source. Alternatively, the composition of gas may be different to the composition of air. This may be desirable, for example, where it is desired to control the exchange of gaseous molecules through the second liquid 2 during use of the microfluidic arrangement. In an example of such an embodiment, the gas comprises less oxygen by volume than air, optionally less than 20% by volume of oxygen, optionally less than 5% by volume of oxygen, optionally less than 1% by volume of oxygen, optionally substantially no oxygen. This may be achieved, for example, by diluting air by adding another gas to air. In an embodiment, the gas is formed by processing air to increase the relative proportion of one or more of the gases in air apart from oxygen. In an embodiment, N₂ is added to air to increase the relative proportion of N₂ in the air and thereby decrease the amount of oxygen. In such an embodiment, the gas comprises more than 80% by volume of nitrogen, optionally more than 85% by volume of nitrogen, optionally more than 90% by volume of nitrogen, optionally more than 95% by volume of nitrogen, optionally substantially 100% nitrogen. In embodiment, CO₂ is added to air to increase the relative proportion of CO₂ in the air and thereby decrease the amount of oxygen. In such an embodiment, the gas comprises more than 0.05% by volume of CO₂, optionally more than 0.1% by volume of CO₂, optionally more than 10% by volume of CO₂, optionally more than 50% by volume of CO₂, optionally substantially 100% CO₂. The molecules introduced into the second liquid 2 by the treatment of the second liquid 2 by flowing gas through the second liquid 2 thus make the relative proportion of oxygen lower in the second liquid 2 than would be the case if the second liquid 2 were saturated by air. If a microfluidic arrangement manufactured in this way is exposed to air for a prolonged period, the treatment can thus hinder net transfer of oxygen into the first liquid 1 in comparison to if the second liquid 2 were not treated. This effect makes it possible to reduce the amount of oxygen supplied through the second liquid 2 to sub-bodies of the first liquid 1 during use, making the environment more similar to conditions within a living body. The microfluidic arrangement can thus be used to perform a biological assay in which exposure of biological matter to molecules from gases is controlled more accurately and flexibly without requiring complex systems for controlling the atmosphere in the environment outside of the microfluidic arrangement.

The time for which the gas needs to be flowed through the second liquid 2 will depend on various factors, including the composition of the gas, the flow rate of the gas, how dispersed the gas bubbles are, the concentration difference between the flowing gas phase and the second liquid 2, the total surface area of the interfaces between the flowing gas phase and the second liquid 2, the diffusion/absorption coefficients, the pressure, and the temperature. If the flow rate is sufficiently high, a significant improvement in performance is seen by flowing the gas through the second liquid 2 for only several seconds (e.g. less than 10 seconds), but lower flow rates may be used and/or the flowing continued for longer periods of time. The improvement in performance can be obtained quickly and reliably. The success of the method is not vulnerable to minor variations in the process, such that time-consuming tuning and/or calibration of the flowing of gas is not essential to achieve acceptable performance.

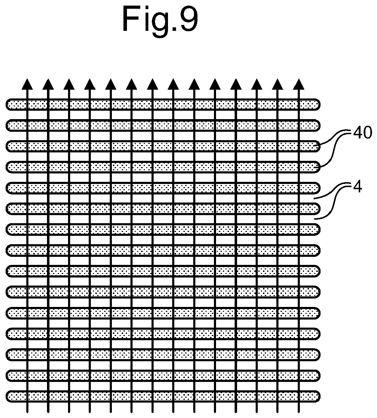

In an embodiment, the continuous body of the first liquid 1 is divided into a plurality of elongate strips 40 (the first liquid 1 in each strip 40 being depicted by hatching for clarity) in an initial step of dividing the continuous body of the first liquid 1 into sub-bodies. In an embodiment, the elongate strips 40 are parallel to each other. An example of such an arrangement is depicted in Figure 9. The arrangement could be formed for example by propelling the separation fluid 3 into contact with the selected path 4 along a series of parallel horizontal trajectories. In a subsequent step, a substance is added to one or more localized regions (e.g. lateral ends) of one or more of the elongate strips 40. The substance migrates (e.g. by diffusion and/or advection) along each elongate strip 40, thereby creating a concentration gradient along the elongate strip 40. In a subsequent step the elongate strips are divided into a plurality of sub-bodies, thereby quickly and easily creating sets of sub-bodies having different concentrations of a selected substance within them. In the particular example of Figure 9, the division of the elongate strips 40 into the plurality of sub-bodies is performed by moving a distal tip 6 of an injection member along the trajectories marked by solid line arrows in Figure 9 while continuously ejecting the separation fluid 3 from the distal tip 6.

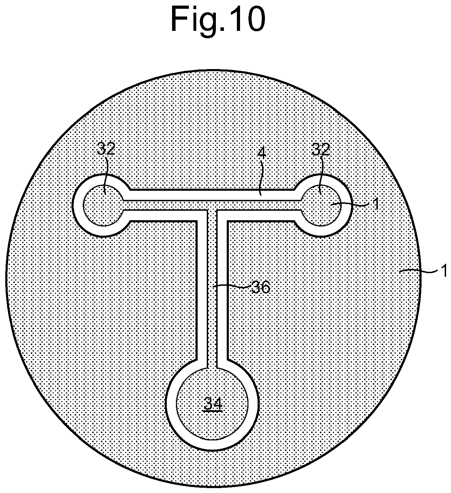

In an embodiment, more complex shapes can be formed by the dividing of the continuous body of the first liquid 1 into sub-bodies. In one example, as depicted in Figure 10 in which regions of the first liquid 1 are hatched for clarity, the continuous body of the first liquid 1 is divided so that at least one sub-body is formed that comprises at least one conduit 36 connected to at least one reservoir 32, 34. The conduit 36 and reservoir 32,34 may be configured so that in use a liquid can be driven through the conduit 36 to or from the reservoir 32,34. The conduit 36 will typically have an elongate form when viewed perpendicularly to the substrate 11. The reservoirs 32, 34 will typically be circular or at least have a lateral dimension that is larger than a width of the conduit 36. In the particular example shown, a T-shaped conduit 36 is provided that connects two source reservoirs 32 and 34 to a sink reservoir 34. Flow is driven in use, e.g. by Laplace pressure, hydrostatic pressure and/or pumping of material into the reservoirs 32, from the source reservoirs 32 to the sink reservoir 34.

Figure 11 depicts an example apparatus 30 for performing methods according to embodiments of the present disclosure. The apparatus 30 is thus configured to manufacture a microfluidic arrangement. The apparatus 30 comprises a substrate table 16. The substrate table 16 holds a substrate 11. A continuous body of first liquid 1 is provided in direct contact with the substrate 11. A second liquid 2 is provided in direct contact with the first liquid 1. The second liquid 2 covers the first liquid 1.

A pattern forming unit is provided that propels a separation fluid 3 through the first liquid 1 and into contact with the substrate 11 along all of the selected path 4. The propulsion of the separation fluid 3 may be performed using any of the methods described above with reference to Figures 1-10.

In the example of Figure 11, the apparatus 30 propels the separation fluid 3 by pumping the separation fluid 3 out of a distal tip 6 of an injection member 15. The apparatus 30 of Figure 11 comprises an injection system. The injection system is configured to pump separation fluid 3 out of the distal tip 6 of the injection member 15. The injection member 15 may comprise a lumen and the separation fluid 3 may be pumped along the lumen to the distal tip 6. In an embodiment, the separation fluid 3 is ejected from the distal tip 6 while the distal tip 6 is moved over the substrate 11 according to the geometry of the selected path 4. The injection system comprises the injection member 15 and a pumping system 17. In use, the pumping system 17 will comprise a reservoir containing the separation fluid 3, conduits for conveying the separation fluid 3 from the reservoir to the lumen of the injection member 15, and a mechanism for pumping the separation fluid 3 through the lumen and out of the distal tip 6 of the injection member 15.

In an embodiment, the apparatus 30 is configured to maintain a small but finite separation between the distal tip 6 of the injection member 15 and the substrate 11 while the injection member 15 is moved over the substrate 11. This is beneficial at least where the microfluidic arrangement is to be used for cell-based studies, which would be affected by any scratching or other modification of the surface that might be caused were the injection member 15 to be dragged over the substrate 11 in contact with the substrate 11. Any such modifications could negatively affect optical access and/or cell compatibility. In an embodiment, this is achieved by mounting the injection member 15 slideably in a mounting such that a force from contact with the substrate 11 will cause the injection member 15 to slide within the mounting. Contact between the injection member 15 and the substrate 11 is detected by detecting sliding of the injection member 15 relative to the mounting. When contact is detected, the injection member 15 is pulled back by a small amount (e.g. 20-150 microns) before the injection member 15 is moved over the substrate 11 (without contacting the substrate 11 during this motion).

The injection system, or an additional injection system configured in a corresponding manner, may additionally provide the initial continuous body of the first liquid 1 in direct contact with the substrate 11 by ejecting the first liquid 1 through a distal tip of an injection member while moving the injection member over the substrate 11 to define the shape of the continuous body of the first liquid 1. In embodiments, the injection system or additional injection system may further be configured to controllably extract the first liquid 1, for example by controllably removing excess first liquid by sucking the liquid back through an injection member.

In the embodiment shown, the pumping system 17 implements the liquid treatment apparatus 50 discussed above, comprising for example components corresponding to the reservoir 52 for holding the second liquid 2 and the pump 54 for pumping gas through the second liquid 2. In an embodiment, the separation fluid 3 consists of the treated second liquid 2 and the same dispensing mechanism is used both to provide the initial layer of second liquid 2 covering the first liquid 1 and to propel the second liquid 2 (acting as the separation fluid 3) onto the substrate 11 to form the isolated sub-bodies 7 of first liquid 1. Functionality corresponding to that provided by the supply conduit 58 of Figure 1 may thus be provided by the injection member 15.

The apparatus 30 of Figure 11 further comprises a controller 10. The controller 10 controls movement of the injection member 15 over the substrate 11 during the propulsion of the separation fluid 3 onto the selected path on the substrate 11 (and, optionally, during forming of the continuous body of the first liquid 1 and/or during dispensing of the second liquid 2). In an embodiment, the apparatus 30 comprises a processing head 20 that supports the injection member 15. The processing head 20 is configured such that the injection member 15 can be selectively advanced and retracted. In an embodiment, the advancement and retraction is controlled by the controller 10, with suitable actuation mechanisms being mounted on the processing head 20. A gantry system 21 is provided to allow the processing head 20 to move as required. In the particular example shown, left-right movement within the page is illustrated but it will be appreciated that the movement can also comprise movement into and out of the page as well as movement towards and away from the substrate 11 (if the movement of the injection member 15 provided by the processing head 20 itself is not sufficiently to provide the required upwards and downwards displacement of the injection member 15).

In some embodiments, a separation fluid 3 is propelled through the first liquid 1 in a continuous process (i.e. without interruption) for at least a portion of the selected path 4. For example, separation fluid 3 may be propelled continuously out of a distal tip 6 of an injection member (e.g. by pumping at a continuous rate) while the distal tip 6 is moved over a portion of the selected path (e.g. in a straight line downwards as depicted in Figure 4 or along one of the vertical solid line arrows in Figure 9). In other embodiments, the propelling of the separation fluid 3 comprises intermittent propulsion of portions of the separation fluid 3 during at least a portion of the displacing of the first liquid 1 away from the selected path 4. For example, the separation fluid 3 may be propelled intermittently during the displacement of the first liquid 1 away from the selected path 4 along the portion of the selected path 4 shown in Figure 4 or along any one of the portions of the selected path represented by the vertical solid line arrows in Figure 9. In such embodiments, the intermittent propulsion may be such that the first liquid 1 is nevertheless displaced away from the selected path 4 so as to cause the selected path 4 to contact the second liquid 2 along a continuous line (e.g. along all of each of one or more of the vertical lines in Figures 4 and 9 referred to above). This may be achieved for example by arranging for different portions of the separation fluid 3 that are intermittently propelled towards the substrate 11 (i.e. propelled at different times relative to each other) to be propelled into contact with the selected path in overlapping regions. Thus, an impact region on the substrate 11 associated with one portion of propelled separation fluid 3 will overlap with the impact region on the substrate 11 associated with at least one other portion of propelled separation fluid 3 (typically propelled at a slightly different time, for example after a head that is driving the propulsion has moved a short distance relative to the substrate 11). The possibility of using intermittent propulsion opens up a wider range of possible mechanisms for driving the propulsion, such as piezoelectric mechanisms.

## Claims

1. A method of manufacturing a microfluidic arrangement, comprising:
providing a continuous body of a first liquid (1) in direct contact with a substrate (11);
providing a second liquid (2) in direct contact with the continuous body of first liquid (1), the second liquid (2) covering the continuous body of first liquid (1); and
causing the second liquid (2) to move through the first liquid (1) and into contact with the substrate (11) along all of a selected path (4) on the surface of the substrate (11), thereby displacing first liquid (1) that was initially in contact with all of the selected path (4) away from the selected path(4), the selected path (4) being such that one or more walls of second liquid (2) are formed that modify a shape of the continuous body of first liquid (1), wherein:
the first liquid (1) is aqueous, and the second liquid (2) is immiscible with the first liquid (1); the method being **characterized in that**:
the second liquid (2) is treated, prior to the providing of the second liquid (2) in direct contact with the continuous body of first liquid (1) and prior to the second liquid (2) being caused to move through the first liquid (1), by flowing a gas through the second liquid (2) and thereby increasing a level of saturation of the second liquid (2).

2. The method of claim 1, wherein the selected path (4) is such that the continuous body of the first liquid (1) is divided to form a single sub-body of first liquid (1) separated from the rest of the continuous body of first liquid (1) by the second liquid (2) or a plurality of sub-bodies of first liquid (1) separated from each other by the second liquid (2).

3. The method of claim 1, wherein the continuous body of first liquid (1) remains a single continuous body of first liquid (1) after the modification of the shape of the continuous body of first liquid (1) by the one or more walls of second liquid (2).

4. The method of any preceding claim, wherein the second liquid (2) is caused to move through the first liquid (1) by propelling a separation fluid (3) through at least the first liquid (1) and into contact with the substrate (11) along all of the selected path (4).

5. The method of claim 4, wherein the separation fluid (3) is propelled onto the selected path (4) on the substrate (11) by pumping the separation fluid (3) from a distal tip (6) of an injection member while providing relative movement between the distal tip (6) and the substrate (11).

6. The method of claim 4 or 5, wherein the separation fluid (3) comprises one or more of the following: a gas, a liquid, a liquid having the same composition as the second liquid (2), a portion of the second liquid (2) provided before the propulsion of the separation fluid (3) through the first liquid (1).

7. The method of any preceding claim, wherein the composition of the gas is different from the composition of air.

8. The method of any preceding claim, wherein the gas comprises less than 20% by volume of oxygen.

9. The method of any preceding claim, wherein the gas is formed by processing air to increase the relative proportion or one or more of the gases in air apart from oxygen.

10. The method of any of claims 1-6, wherein the gas consists of air.

11. The method of any preceding claim, wherein the first liquid (1) comprises a cell culture medium.

12. The method of any preceding claim, wherein the second liquid (2) comprises a fluorocarbon.

## Patentansprüche

1. Verfahren zur Herstellung einer mikrofluidischen Anordnung, das Folgendes umfasst:
Bereitstellen eines kontinuierlichen Körpers aus einer ersten Flüssigkeit (1) in direktem Kontakt mit einem Substrat (11);
Bereitstellen einer zweiten Flüssigkeit (2) in direktem Kontakt mit dem kontinuierlichen Körper der ersten Flüssigkeit (1), wobei die zweite Flüssigkeit (2) den kontinuierlichen Körper der ersten Flüssigkeit (1) bedeckt; und
Bewirken, dass sich die zweite Flüssigkeit (2) durch die erste Flüssigkeit (1) und in Kontakt mit dem Substrat (11) entlang eines gesamten ausgewählten Pfades (4) auf der Oberfläche des Substrats (11) bewegt, wodurch die erste Flüssigkeit (1), die ursprünglich mit dem gesamten ausgewählten Pfad (4) in Kontakt war, von dem ausgewählten Pfad (4) weg verdrängt wird, wobei der ausgewählte Pfad (4) so beschaffen ist, dass eine oder mehrere Wände der zweiten Flüssigkeit (2) gebildet werden, die eine Form des kontinuierlichen Körpers der ersten Flüssigkeit (1) modifizieren, wobei:
die erste Flüssigkeit (1) wässrig ist, und die zweite Flüssigkeit (2) mit der ersten Flüssigkeit (1) nicht mischbar ist;
wobei das Verfahren **dadurch gekennzeichnet ist, dass**:
vor dem Bereitstellen der zweiten Flüssigkeit (2) in direktem Kontakt mit dem kontinuierlichen Körper der ersten Flüssigkeit (1) und bevor die zweite Flüssigkeit (2) dazu gebracht wird, sich durch die erste Flüssigkeit (1) zu bewegen, die zweite Flüssigkeit (2) behandelt wird, indem ein Gas durch die zweite Flüssigkeit (2) geleitet wird, wodurch der Sättigungsgrad der zweiten Flüssigkeit (2) erhöht wird.

2. Verfahren nach Anspruch 1, wobei der gewählte Pfad (4) so beschaffen ist, dass der kontinuierliche Körper der ersten Flüssigkeit (1) geteilt wird, um einen einzigen Teilkörper der ersten Flüssigkeit (1) zu bilden, der vom Rest des kontinuierlichen Körpers der ersten Flüssigkeit (1) durch die zweite Flüssigkeit (2) getrennt ist, oder eine Vielzahl von Teilkörpern der ersten Flüssigkeit (1) zu bilden, die durch die zweite Flüssigkeit (2) voneinander getrennt sind.

3. Verfahren nach Anspruch 1, wobei der kontinuierliche Körper der ersten Flüssigkeit (1) nach der Veränderung der Form des kontinuierlichen Körpers der ersten Flüssigkeit (1) durch die eine oder mehrere Wände der zweiten Flüssigkeit (2) ein einzelner kontinuierlicher Körper der ersten Flüssigkeit (1) bleibt.

4. Verfahren nach einem der vorhergehenden Ansprüche, wobei die zweite Flüssigkeit (2) veranlaßt wird, sich durch die erste Flüssigkeit (1) zu bewegen, indem ein Trennfluid (3) zumindest durch die erste Flüssigkeit (1) und in Kontakt mit dem Substrat (11) entlang des gesamten ausgewählten Pfades (4) getrieben wird.

5. Verfahren nach Anspruch 4, wobei das Trennfluid (3) auf den ausgewählten Pfad (4) auf dem Substrat (11) getrieben wird, indem das Trennfluid (3) von einer distalen Spitze (6) eines Injektionselements gepumpt wird, wobei eine relative Bewegung zwischen der distalen Spitze (6) und dem Substrat (11) vorgesehen ist.

6. Verfahren nach Anspruch 4 oder 5, wobei das Trennfluid (3) eines oder mehrere von Folgendem umfasst: ein Gas, eine Flüssigkeit, eine Flüssigkeit mit der gleichen Zusammensetzung wie die zweite Flüssigkeit (2), einen Teil der zweiten Flüssigkeit (2), der vor dem Vortrieb des Trennfluids (3) durch die erste Flüssigkeit (1) bereitgestellt wird.

7. Verfahren nach einem der vorhergehenden Ansprüche, wobei die Zusammensetzung des Gases von der Zusammensetzung der Luft verschieden ist.

8. Verfahren nach einem der vorhergehenden Ansprüche, wobei das Gas weniger als 20 Volumenprozent Sauerstoff enthält.

9. Verfahren nach einem der vorhergehenden Ansprüche, wobei das Gas durch Aufbereitung von Luft gebildet wird, um den relativen Anteil eines oder mehrerer Gase in der Luft außer Sauerstoff zu erhöhen.

10. Verfahren nach einem der Ansprüche 1 bis 6, wobei das Gas aus Luft besteht.

11. Verfahren nach einem der vorhergehenden Ansprüche, wobei die erste Flüssigkeit (1) ein Zellkulturmedium umfasst.

12. Verfahren nach einem der vorhergehenden Ansprüche, wobei die zweite Flüssigkeit (2) einen Fluorkohlenstoff umfasst.

## Revendications

1. Procédé de fabrication d'un agencement microfluidique, comprenant :
la fourniture d'un corps continu d'un premier liquide (1) en contact direct avec un substrat (11) ;
la fourniture un second liquide (2) en contact direct avec le corps continu du premier liquide (1), le second liquide (2) recouvrant le corps continu du premier liquide (1) ; et
le déplacement du second liquide (2) à travers le premier liquide (1) et en contact avec le substrat (11) le long de tout un trajet sélectionné (4) sur la surface du substrat (11), déplaçant ainsi le premier liquide (1) qui était initialement en contact avec l'ensemble du trajet sélectionné (4) loin du trajet sélectionné (4), le trajet sélectionné (4) étant tel qu'une ou plusieurs parois du second liquide (2) qui modifient une forme du corps continu du premier liquide (1) se forment,
dans lequel :
le premier liquide (1) est aqueux, et le second liquide (2) est non miscible avec le premier liquide (1) ;
le procédé étant **caractérisé en ce que** :
le second liquide (2) est traité, avant la fourniture du second liquide (2) en contact direct avec le corps continu du premier liquide (1) et avant que le second liquide (2) soit amené à se déplacer à travers le premier liquide (1), en faisant circuler un gaz à travers le second liquide (2) et en augmentant ainsi un niveau de saturation du second liquide (2).

2. Procédé selon la revendication 1, dans lequel le trajet sélectionné (4) est tel que le corps continu du premier liquide (1) est divisé pour former un seul sous-corps du premier liquide (1) séparé du reste du corps continu du premier liquide (1) par le second liquide (2) ou une pluralité de sous-corps du premier liquide (1) séparés les uns des autres par le second liquide (2).

3. Procédé selon la revendication 1, dans lequel le corps continu du premier liquide (1) reste un seul corps continu du premier liquide (1) après la modification de la forme du corps continu du premier liquide (1) par l'une ou plusieurs parois du second liquide (2).

4. Procédé selon l'une quelconque des revendications précédentes, dans lequel le second liquide (2) est amené à se déplacer à travers le premier liquide (1) en propulsant un fluide de séparation (3) à travers au moins le premier liquide (1) et en contact avec le substrat (11) le long de tout le trajet sélectionné (4).

5. Procédé selon la revendication 4, dans lequel le fluide de séparation (3) est propulsé sur le trajet sélectionné (4) sur le substrat (11) en pompant le fluide de séparation (3) à partir d'une pointe distale (6) d'un élément d'injection tout en fournissant un mouvement relatif entre la pointe distale (6) et le substrat (11).

6. Procédé selon la revendication 4 ou 5, dans lequel le fluide de séparation (3) comprend un ou plusieurs des éléments suivants : un gaz, un liquide, un liquide ayant la même composition que le second liquide (2), une partie du second liquide (2) prévue avant la propulsion du fluide de séparation (3) à travers le premier liquide (1).

7. Procédé selon l'une quelconque des revendications précédentes, dans lequel la composition du gaz est différente de la composition de l'air.

8. Procédé selon l'une quelconque des revendications précédentes, dans lequel le gaz comprend moins de 20 % en volume d'oxygène.

9. Procédé selon l'une quelconque des revendications précédentes, dans lequel le gaz est formé en traitant de l'air pour augmenter la proportion relative d'un ou plusieurs des gaz dans l'air en dehors de l'oxygène.

10. Procédé selon l'une quelconque des revendications 1 à 6, dans lequel le gaz est constitué d'air.

11. Procédé selon l'une quelconque des revendications précédentes, dans lequel le premier liquide (1) comprend un milieu de culture cellulaire.

12. Procédé selon l'une quelconque des revendications précédentes, dans lequel le second liquide (2) comprend un fluorocarbone.
